# EUROPEAN PATENT SPECIFICATION

(11) **EP 4 122 523 B1**
(45) Date of publication and mention of the grant of the patent: **02.12.2026**
(21) Application number: 22195818.4
(22) Date of filing: 12.08.2011
(51) Int. Cl.: A61M 25/14, A61M 25/00

(54) **TRIMMABLE CATHETER INCLUDING DISTAL PORTION STABILITY FEATURES**
EINSTELLBARER KATHETER MIT STABILITÄTSFUNKTIONEN DES DISTALEN TEILS
CATHÉTER AJUSTABLE PRÉSENTANT DES CARACTÉRISTIQUES DE STABILISATION DE SA PARTIE DISTALE

(30) Priority: 12.08.2010 US 37300010 P
(43) Date of publication of application: 25.01.2023
(62) Document number(s) of the earlier application(s) in accordance with Art. 76 EPC: 11817136.2 / 2 603 275
(73) Proprietor: C. R. Bard, Inc., Franklin Lakes, NJ 07417 (US)
(72) Inventor: Blanchard, Daniel B., Bountiful, UT 84010 (US); Patterson, Ryan C., Farmington, UT 84025 (US)
(74) Representative: Hoffmann Eitle

(56) References cited:
- WO-A1-2009/131583
- WO-A1-2009/142904
- WO-A1-2010/054666
- WO-A1-96/06653
- WO-A1-96/39217
- US-A1- 2004 199 192
- US-A1- 2006 004 316

## Description

### CROSS-REFERENCE TO RELATED APPLICATIONS

This application claims the benefit of U.S. Provisional Patent Application No. 61/373,000, filed August 12, 2010, and entitled "Trimmable Catheter Including a Flared Distal Portion".

### PRIOR ART

WO2009/142904 **describes a catheter assembly having a conical diffuser tip for use in rapid infusion procedures. The conical diffuser tip provides a flared opening whereby the velocity of an infusant is decreased as the infusant travels through the catheter tip and exits into the vascular system of a patient. This decrease in velocity proportionately reduces the backpressure and/or recoil force of the catheter assembly thereby permitting the use of higher infusion rates.**
WO2010/054666 **describes a catheter comprising a first tubular catheter part extending longitudinally between a proximal end provided with at least one inlet, and a distal end provided with at least one outlet and a second tubular catheter part extending longitudinally between a proximal end provided with one opening and a distal end provided with a second opening. The first tubular catheter part is longer than the second tubular catheter part. The second tubular catheter part is arranged on the first tubular catheter at the distal end and the first tubular catheter part and the second tubular catheter part are permanently displaceable fixed relative to each other. This provides a catheter which has distal end which is more stable than conventional intermittent catheters and thus is easier to insert with a reduced risk that the catheter bends or kinks during catherisation.**
US2006/004316 **describes a catheter tip designed to reduce the outflow velocity and/or directional momentum of fluid being infused by a catheter having such a tip. In one variation, a plurality of channels is provided at the distal portion of the catheter to increase the outflow cross-sectional area. In another variation, the diameter of the catheter at its distal portion where the fluid exits is increased. In yet another variation, a bullet-shaped nose is implemented which may decrease turbulence at the distal end of catheter tip. The low velocity outflow catheter tip may also be implemented on a dual lumen catheter, such as a hemodialysis catheter, to reduce circulation rate.**
US2004/199192 **describes a phacoemulsification needle comprising a shaft, a tip disposed at a distal end of the shaft, the tip having a larger outer diameter than the shaft and an aspiration lumen extending through the shaft and the tip. The tip has an opening communicating with the aspiration lumen, a ball-shaped surface and a flat distal end comprising the opening. The needle can be used for removal of the cataract nucleus as well as for removal of the residual tissue.**
WO96/39217 **describes an exchange catheter which facilitates the replacement of an existing guiding catheter with a larger guiding catheter during catheterisation procedures such as percutaneous transluminal coronary angioplasty without disturbing the position of a guidewire. The exchange catheter has a distal portion through which a guidewire is slidable. The outer diameter of the distal portion is slightly less than the inner diameter of the existing guiding catheter. A femoral sheath is slidable over the proximal portion of the exchange catheter so as to facilitate femoral sheath exchange.**
WO96/06653 **describes a catheter having a flexible tip portion. A plurality of regions of a first transverse rigidity are formed in the flexible tip portion. The catheter has a second transverse rigidity along its length which is greater than the first transverse rigidity. The regions of the lesser first transverse rigidity are formed by areas of the catheter tip having an average transverse cross-sectional area less than a transverse cross-sectional area of the remainder of the catheter apparatus. The plurality of first transverse rigidity regions are formed by a respective plurality of circumferential cutout regions in the catheter tip.**
WO2009/131583 **describes a vascular access catheter that has a catheter shaft with a distal portion having a distal end portion with a distal tip having a sloped face that is positioned at an acute angle from the distal tip relative to a longitudinal axis of the catheter shaft. A first, second and third lumen extend longitudinally through the catheter shaft. The third lumen is configured for receiving a guidewire and may extend a partial length of the catheter or substantially the entire length of the catheter and may have a port defined therein the outer surface of the catheter shaft that it is in communication with. The first lumen has an aperture located in the angled edge distal end portion of the catheter next to the distal tip and communicates with the first lumen.**

### BRIEF SUMMARY

Briefly summarized, embodiments of the present invention are directed to a catheter assembly used to gain access to a vasculature or other internal portion of a patient. The catheter assembly includes a catheter tube that defines one or more lumens, with at least one lumen optionally capable of power injection, in one embodiment. A proximal portion of the catheter tube is sized and configured so as to reside within a portion of the vessel that is disposed relatively close to the insertion site of the catheter tube into the patient. In particular, as such portions of the vessel are of relatively small diameter, the proximal portion of the catheter tube is similarly of relatively small diameter and of relatively low stiffness so as to prevent substantial occlusion of the vessel by the catheter tube and lessen vessel damage.

Moreover, a distal portion of the catheter tube is sized and configured such that the distal portion remains stable within the vessel during infusion of fluids therethrough. Particularly, the distal portion of the catheter tube is configured to avoid whipping of the distal tip within the vessel during fluid infusion so as to prevent vessel damage. This stability of the catheter tube's distal portion is especially helpful during power injection of fluids into the vessel.

In one embodiment, stability of the distal tip of the catheter tube is accomplished by flaring, or increasing the cross sectional size of the distal portion of the tube, thus increasing the areal size of the one or more catheter lumens and improving the area moment of inertia of the distal portion. In another embodiment, the elastic modulus, or stiffness, of the distal portion can be increased relative to the proximal portion of the catheter tube. In another embodiment, both the area moment of inertia and the modulus can be modified to enhance distal portion stability. Note that these and related parameters can be modified in other ways as well.

According to the present invention, there is provided a catheter system comprising the features of claim 1. Preferred embodiments of the present invention are defined in the dependent claims.

These and other features of embodiments of the present invention will become more fully apparent from the following description and appended claims, or may be learned by the practice of embodiments of the invention as set forth hereinafter.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 is a perspective view of a catheter assembly configured in accordance with one claimed embodiment;
FIG. 2 is a side view of the catheter tube of the assembly shown in FIG. 1;
FIGS. 3A-3C are cross sectional views of the catheter tube of FIG. 2, showing a relative change in catheter tube structure;
FIG. 4 is a side view of a catheter tube configured in accordance with one claimed embodiment;
FIG. 5 is a side view of a catheter tube configured in accordance with one claimed embodiment;
FIG. 6 is a side view of a catheter tube configured in accordance with one claimed embodiment;
FIG. 7 is a cross sectional view of a distal portion of a catheter tube in accordance with one claimed embodiment;
FIG. 8 is a side view of a catheter tube in accordance with one unclaimed example;
FIG. 9 is a side view of a catheter tube in accordance with an unclaimed example;
FIG. 10 is a side view of a catheter tube in accordance with an unclaimed example;
FIGS. 11A and 11B are cross sectional views of the catheter tube of FIG. 10;
FIG. 12 is a cross sectional side view of a distal portion of a catheter tube in accordance with one unclaimed example;
FIG. 13 is a perspective view of a distal portion of a catheter tube in accordance with one unclaimed example;
FIGS. 14A and 14B are cross sectional side views of a catheter tube in accordance with one claimed embodiment;
FIG. 15 is a cross sectional side view of a catheter tube in accordance with one claimed embodiment;
FIG. 16 is a cross sectional side view of a catheter tube in accordance with one claimed embodiment;
FIG. 17 is a cross sectional side view of a catheter tube in accordance with one unclaimed example;
FIG. 18 is a cross sectional side view of a catheter tube in accordance with one unclaimed example;
FIG. 19 is a cross sectional side view of a catheter tube in accordance with one unclaimed example;
FIG. 20 is a cross sectional side view of a catheter tube in accordance with one unclaimed example;
FIG. 21 is a cross sectional side view of a catheter tube in accordance with one unclaimed example;
FIG. 22 is a side view of a catheter tube in accordance with one unclaimed example;
FIGS. 23A-23C are cross sectional views of the catheter tube of FIG. 22;
FIG. 24 is a side view of a catheter tube in accordance with one claimed embodiment;
FIG. 25 is a distal end view of the catheter tube of FIG. 24;
FIG. 26 is a distal end view of the catheter tube of FIG. 24 in a first rolled-up configuration;
FIG. 27 is a distal end view of the catheter tube of FIG. 24 in a second rolled-up configuration; and
FIGS. 28A and 28B are perspective and end views, respectively, of a catheter tube in accordance with one claimed embodiment.

### DETAILED DESCRIPTION OF SELECTED EMBODIMENTS

Reference will now be made to figures wherein like structures will be provided with like reference designations. It is understood that the drawings are diagrammatic and schematic representations of unclaimed examples and claimed exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a catheter placed within the body of a patient is considered a distal end of the catheter, while the catheter end remaining outside the body is a proximal end of the catheter. Also, the words "including," "has," and "having," as used herein, including the claims, shall have the same meaning as the word "comprising."

Embodiments of the present invention are generally directed to a catheter assembly used to gain access to a vasculature or other internal portion of a patient. The catheter assembly includes a catheter tube that defines one or more lumens, with at least one lumen capable of power injection, in one embodiment. A proximal portion of the catheter tube is sized and configured so as to reside within a portion of the vessel that is disposed relatively close to the insertion site of the catheter tube into the patient. In particular, as such portions of the vessel are of relatively small diameter, the proximal portion of the catheter tube is similarly of relatively small diameter and of relatively low stiffness so as to enable ease of passage of the catheter tube through the vessel and to prevent substantial occlusion of the vessel by the catheter tube.

Moreover, a distal portion of the catheter tube is sized and configured such that it remains stable within the vessel during infusion of fluids therethrough. Particularly, the distal portion of the catheter tube is configured to avoid whipping of the distal tip within the vessel during fluid infusion so as to prevent vessel damage. This stability of the catheter tube's distal portion is especially helpful during power injection of fluids into the vessel.

In one embodiment, stability of the distal tip of the catheter tube is accomplished by flaring, or increasing the cross sectional size of the distal portion of the tube thus increasing the areal size of the one or more catheter lumens and improving the moment area of inertia of the distal portion. In another embodiment, the elastic modulus, or stiffness, of the distal portion can be increased relative to the proximal portion of the catheter tube. In another embodiment, both the moment area of inertia and the modulus can be modified to enhance distal portion stability. Note that these and related parameters can be modified in other ways as well and that additional configurations for increasing distal tip stability are disclosed. In addition to the catheter tubes described herein as part of catheter assemblies, the principles to be disclosed can be employed with other tubular medical devices as well.

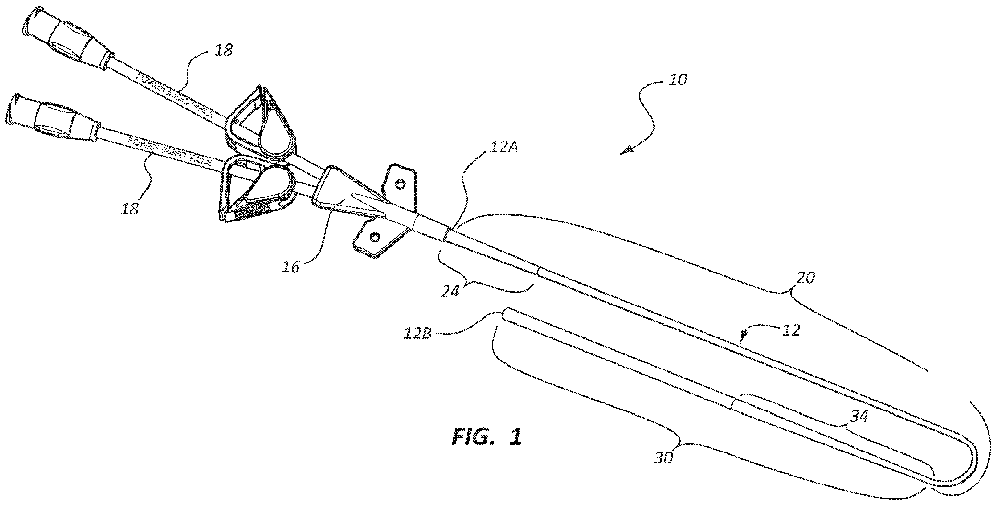

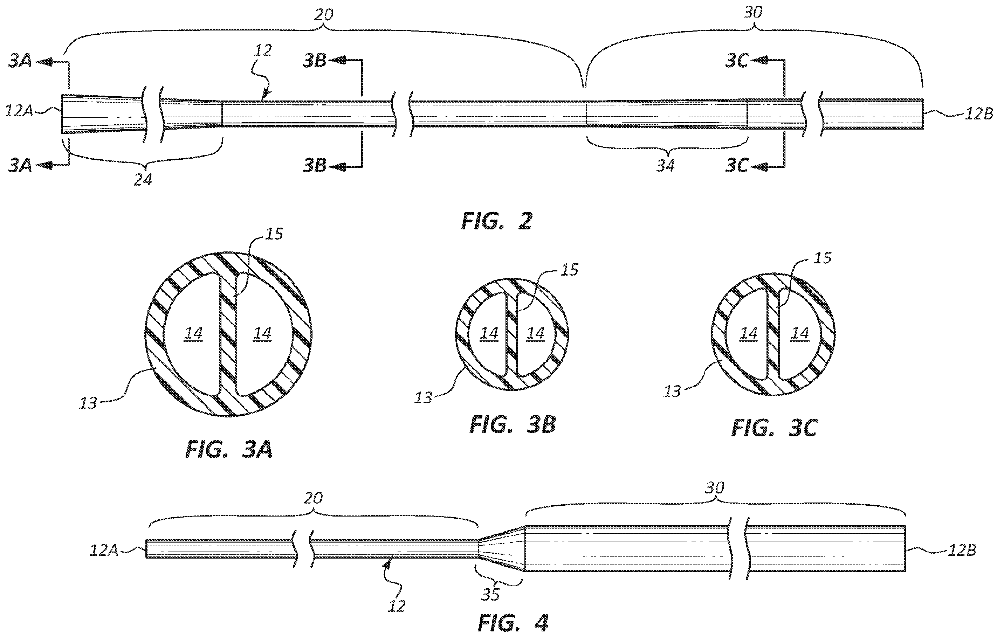

Reference is first made to FIG. 1, which depicts various details of a catheter assembly, generally designated at 10, according to one embodiment. As shown, the catheter assembly ("catheter") 10 includes an elongate catheter tube 12 formed by an outer wall 13 which, together with a septum 15 (FIGS. 3A-3C) defines one or more lumens 14 extending between a proximal end 12A and a distal end 12B of the tube. A bifurcation 16 mates with the catheter tube 12 at the proximal end 12A thereof to provide fluid communication between the catheter tube and one or more extension legs 18.

FIG. 2 shows further details of the catheter tube of the catheter 10, according to the present embodiment. As shown, the tube 12 includes a proximal portion 20 extending distally from the proximal end 12A and a distal portion 30 extending distally from the distal end of the proximal portion to the distal end 12B of the tube. A tapered region 24 of the proximal portion 20 is included in the catheter tube 12 and is configured such that the thickness of the outer wall 13 and septum 15 decrease from the tube distal end 12A to the distal termination of the tapered region 24. In addition, the areal size of each of the lumens 14 also decreases distally over this region. These size differences can be seen by comparing FIGS. 3A and 3B, which show cross sectional views of the catheter tube 12 at the proximal end of the tapered region 24 (FIG. 3A) and distal to the distal termination of the tapered region (FIG. 3B).

The proximal tapered region 24 provides sufficient outer wall thickness and rigidity for the catheter tube 12 of the present embodiment so as to prevent kinking and to enable the tube to be adjusted with respect to an insertion site in the skin of the patient during insertion and adjustment of the catheter 10. Furthermore, the proximal tapered region 24 serves to plug the insertion site through which the catheter tube passes into the patient's body, thus reducing bleeding or other complications at the site.

The remainder of the proximal portion 20 of the catheter tube 12 resides within distal portions of the vessel that are typically located relatively close to the catheter insertion site in extremities of the patient, such as the arm. Such outlying, or distal, vessels are relatively smaller than the larger vessels located deeper within the body in which the distal portion 30 of the catheter tube 12 is disposed. Thus, the proximal portion 20 of the catheter tube 12 distal to the tapered region 24 is similarly sized relatively small so that this portion can reside in the relatively small vessel without occluding a significant portion thereof, which in turn reduces the risk of thrombus. Further, the smaller size of the proximal portion 20 enables it to bend more easily during insertion into the vessel along a potentially tortuous path, resulting in less trauma and damage to the vessel.

As best seen in FIG. 2, the distal portion 30 of the catheter tube 12 includes a distal taper region 34 that provides a size transition for the tube from the distal end of the proximal portion 20 and the remainder of the distal portion 30. Specifically, the distal taper region 34 enables the dimensions of the catheter tube 12 to change from the cross sectional configuration corresponding to the proximal portion 20 shown in FIG. 3B to that of the distal portion 30 shown in FIG. 3C, wherein the thickness of the outer wall 13 and septum 15, together with the areal size of the lumens 14, increase. This general size increase of the catheter tube distal portion 30 provides enhanced stability for this portion of the catheter tube 12, which in turn prevents oscillatory movement, or whipping, of the distal tip of the tube during fluid infusion through the catheter into the vessel in which the catheter 10 is disposed. This in turn reduces the chance for vessel wall damage caused by repetitive impacts of the whipping catheter tube.

In greater detail, it is appreciated that an unsupported stable length, L, of a distal portion of a catheter tube can be characterized by: $L = \frac{c}{Q} \sqrt{\frac{EIA}{ρ}} ,$ (1) where ρ is the density of the injected fluid, Q is the flow rate of the injected fluid, A is the cross sectional lumen area of the catheter tube, E is the elastic modulus of the catheter tube material from which the outer wall 13 and septum 15 are formed, I is the area moment of inertia of the outer wall and septum material, and c is a constants of proportionality.

From equation (1), it is seen that the stability length L of the catheter tube 12 can be increased by increasing one or more of the parameters E, I, and A. The increased size of the distal portion 30 of the catheter tube 12 and the portions of the lumens 14 it defines (FIGS. 1 and 2) serves to increase both the area moment of inertia I and the lumen area A, which in turn improves the stability length L, which corresponds to a more stable tip within the vessel during fluid injection. This tip stability of the distal portion 30 of the catheter tube 12 is especially helpful during power injection of fluids into the vessel, where catheter fluid flow rate Q can exceed about 5 cc/second. Under such conditions, the ability to preserve tip stability and reduce or eliminate tip whipping is especially beneficial.

As discussed above, an increase in the size of the distal portion 30 and/or other portions of the catheter tube 12 increases the area moment of inertia I and thus improves tip stability. Note that I is directly related by the fourth power of the radius of the catheter tube. As such, a relatively small increase in the cross sectional size of the catheter tube can have a significant effect on I, which enhances distal tip stability. Further note that I can be beneficially improved by increasing the thickness of the outer wall and/or septum of the catheter tube while not increasing the area A of the lumen(s) of the tube.

Also, the flexural stiffness, defined as the product of the parameters E and I, is higher in the distal portion 30 relative that of the proximal portion 20, in one embodiment, in order to provide distal tip stability during fluid infusion. This can be accomplished by increasing the elastic modulus and/or the area moment of inertia for the distal portion over that of the proximal portion in any one of the ways discussed herein, or in other ways contemplated by one skilled in the art.

Again as discussed above, an increase in the size of the distal portion 30 and/or other portions of the catheter tube 12 increases the area A of the lumens 14 and thus improves tip stability. Note that in other embodiments lumen area A can be desirably increased in other ways as well, including: a thinning of the outer wall and septum while taking care not to reduce the area moment of inertia A of the distal portion; increasing the lumen area while maintaining the outer diameter of the catheter tube constant, etc.

Though it can be formed to a variety of lengths to suit different uses and applications, in one embodiment the proximal taper region 24 of the proximal portion 20 is about 4 cm in length, the remainder of the proximal portion is about 20 to about 25 cm, the distal taper region 34 of the distal portion 30 is about 4 cm, and the remainder of the distal portion is about 35 cm. Of course, other lengths for the various segments described above can be employed.

Moreover, the lengths, cross sectional sizes of, and relative size differences between the various segments can also vary from what is shown and described herein, as appreciated by one skilled in the art. In the present embodiment, the approximate diameter, wall thickness, and septum thickness for each of the cross section views shown in FIGS. 3A-3C are, respectively: 2.413mm, 0.2794mm, 0.2032mm (.095, .011, .008 in inches) (FIG. 3A); 1.6mm, 0.1778mm, 0.14mm (.063, .0070, .0055 in inches) (FIG. 3B); and 1.778mm, 0.2032mm, 0.1524mm (.070, .0080, .0060 in inches) (FIG. 3C). Note that these are but one possible example of size configurations for the catheter tube 12. Indeed a variety of sizes, including various French sizes, can be employed for the catheter tube. In one embodiment, for example, the proximal catheter portion defines a size in the range of 2-6 French while the distal portion defines a size in the range of 2.5-8 French. It should be further appreciated that the number, size, and other configuration of the catheter lumens can vary from what is shown here. For instance, principles of the present disclosure can be employed with triple and quad lumen catheters. In addition, though the lumens 14 shown in FIGS.3A-3C are symmetrically arranged, in another embodiment the lumens can be included in an offset configuration so as to provide one or more relatively larger lumens for power injection, if desired.

In this and various other embodiments described herein the catheter tube 12 is extruded or otherwise formed from one or more of a variety of suitable materials, including thermoplastic polyurethanes sold under the trademarks TECOFLEX (type 93AB30) and TECOTHANE (type 95A) of Lubrizol Advanced Materials, Inc., and CARBOTHANE (type 95A), the thermoplastic elastomer sold under the trademark PEBAX of Arkema, Inc., silicone, polyesters, polyethylene, etc. Other suitable materials may also be acceptably used, as appreciated by one skilled in the art.

Note that, though the distal portion of the catheters described here are trimmable, it is desirable that the length of the distal portion remaining after trimming is at least as long as the unsupported stable length L as determined by equation (1), above. In one embodiment, this length is from about 3 to about 10 cm, though other stable lengths are possible, per equation (1).

FIG. 4 shows the catheter tube 12 according to another embodiment, including as before the proximal portion 20 and the distal portion 30 joined by a taper region 35. As shown, the distal portion 30 is flared in size from that of the proximal portion 20, thus providing for enhanced tip stability during power injection or other fluid infusion into the vessel. Note further that, whereas the catheter tube design of FIG. 2 provides for distal trimming of the tube, the catheter tube of FIG. 4 is both proximally and distally trimmable so as to adjust the tube length to the anatomy of the patient. Note that in one embodiment the amount trimmed from the distal end 12B of the catheter tube 12 of FIG. 4 should be such that the remaining portion of the flared distal portion 30 is sufficiently long to ensure distal tip stability during fluid infusion, as suggested by equation (1).

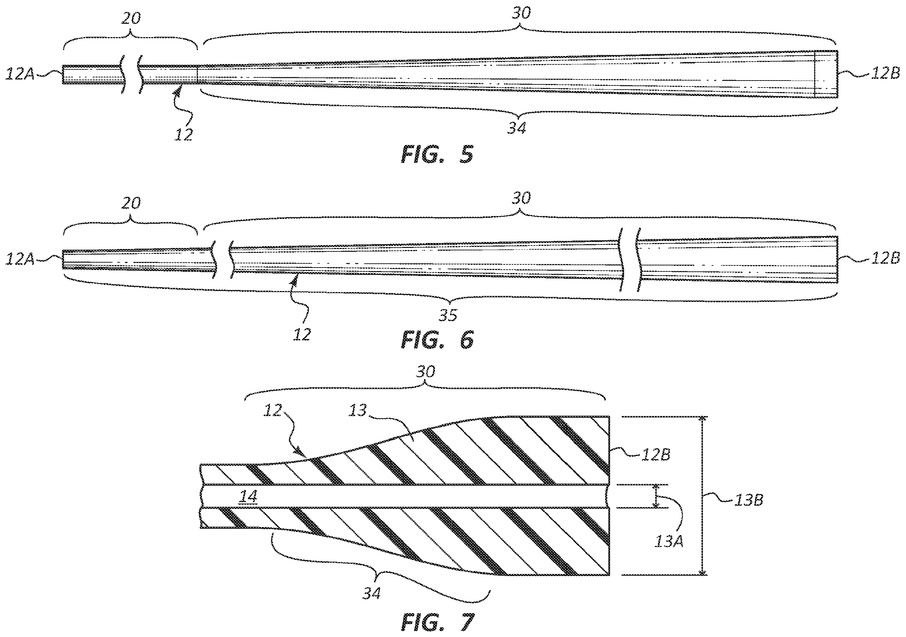

FIGS. 5 and 6 give further possible examples of flared catheter tubes 12, wherein the tube of FIG. 5 includes a relatively short proximal portion 20 and a relatively long distal portion 30 extending to the distal tube end 12B. As shown, in this embodiment the entirety of the distal portion 30 serves as the distal taper portion 34 such that the cross sectional size of the distal portion steadily increases toward the distal end of the tube.

In FIG. 6, the taper region 35 extends along the entirety of the length of the catheter tube 12 such that the entire tube includes a taper. As such, both the proximal portion 20 and the distal portion 30 are tapered, or flared. As before, the tapering disclosed in FIGS. 5 and 6 provides for a relatively small and flexible proximal portion suitable for placement in smaller portions of the vessel relatively close to the catheter insertion site, while also providing a stable distal portion that reduced or eliminates distal tip whipping during power injection or other fluid infusion through the distal end of the catheter tube. Such catheter tubes as those described here in connection with FIGS. 5 and 6 or in other embodiments herein can include one, two, or more lumens. Note further that the catheter tube embodiments shown in FIGS. 5 and 6 and in various other embodiments herein are distally trimmable to suit the vasculature of the patient.

With reference to FIGS. 2 and 3C, note that in one embodiment a relationship between the thickness of the outer wall 13 and the cross sectional radius in the distal portion 30 of the catheter tube 12 is established, wherein the outer wall thickness equals about 0.24 multiplied by the outer radius of the catheter tube in the distal portion. So configured, the distal portion 30 offers enhanced distal tip stability during power injection. Put another way, for a single lumen power injectable catheter tube the product of I and A (and hence tube stability) can be maximized where the outer wall thickness of the catheter tube equals about 0.24 of the catheter tube radius in the distal portion thereof. This relationship can be extrapolated for multi-lumen catheter tubes as well. This relationship can be also employed in a catheter tube regardless of whether the distal portion thereof is flared as shown in FIGS. 2, 4, 5, 6, etc. In another embodiment, the outer wall thickness can equal the product of the outer radius of the catheter tube and a number within a range of from about 0.2 to about 0.3.

It is further appreciated that other catheter factors can be adjusted to maximize distal tip stability for the catheter tube 12, including the length of the flared distal portion alone and as a function of overall catheter tube length, the density of the catheter tube material, the degree of flare of the distal portion with respect to non-flared tube portions, the interaction of modulus, area moment of inertia, and lumen area in the flared portion, etc.

FIG. 7 shows a single lumen the catheter tube 12 according to one embodiment, wherein the distal portion 30 includes the distal taper portion 34 such that a flared outer diameter 13B is defined by the outer wall 13. An inner diameter 13A defined by the lumen 14 remains substantially constant through the distal portion 30. The resulting increase in thickness of the outer wall provides stability for the distal portion 30 during fluid infusion into the vessel, thus reducing or preventing undesired distal tip whipping.

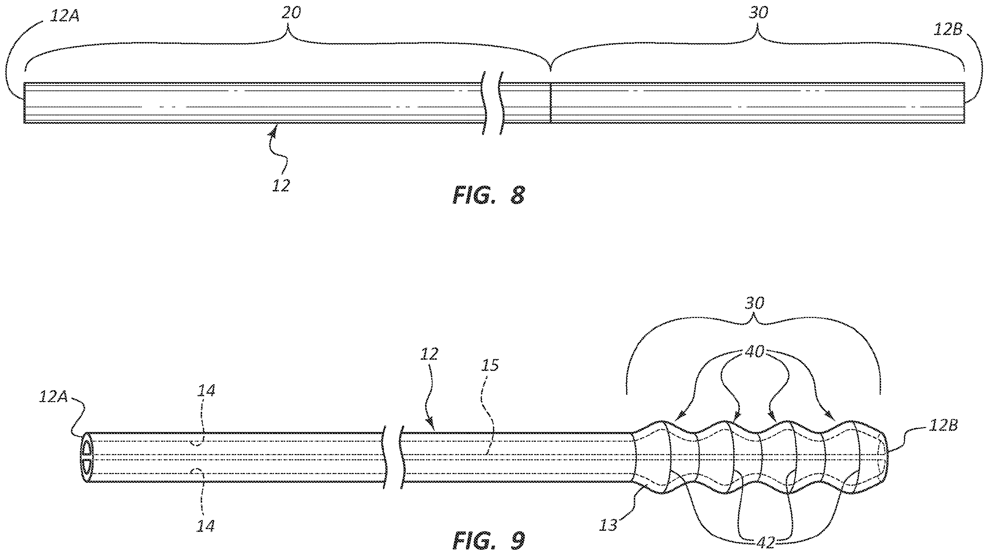

FIG. 8 shows that, in one embodiment, the proximal portion 20 and the distal portion 30 of the catheter tube 12 can be configured so as to exhibit differing levels of the stiffness, or elastic modulus, E *(see* equation (1)). For instance, in the present embodiment the proximal portion 20 includes a relatively soft material while the distal portion 30 includes a relatively stiffer material so as to provide extra strength at the distal end in order to increase the elastic modulus E of the distal portion, in turn reducing or preventing distal tip whipping. In yet another embodiment, the proximal and distal portions can exhibit similar stiffness at room temperature, but exhibit differing stiffness after implantation and subjection to internal body temperatures.

In one embodiment, both the proximal and distal portions are formed of a similar material, with the distal portion being treated to be stiffer relative the proximal portion. Examples of such treatment of the distal portion include irradiation, application of a solvent or activator, heat treatment, etc. In another embodiment, it is the proximal portion that is treated to exhibit a less-stiff elastic modulus.

In one possible embodiment, the entire length of the catheter tube can be treated so as to exhibit a relatively stiff modulus. In another embodiment, the catheter tube can be extruded from two different materials to provide a soft proximal portion and a relatively stiffer distal portion. In yet another embodiment, a soft proximal tube portion can be formed then joined via adhesion to a preformed, relatively stiffer distal portion. These and other variations are therefore contemplated.

FIG. 9 shows details of the catheter tube 12 according to a non-claimed example, wherein the distal portion 30 of the tube outer wall 13 defines a plurality of flared segments 40 that each include an annular maximum diameter point 42. The catheter tube 12 here is a dual lumen tube with the lumens 14 separated by the septum 15. The plurality of flared segments gives the catheter tube 12 a knurled appearance. The annular flared segments 40 are configured such that any one of the segments can be cut at about the respective maximum diameter point 42, thus shortening the catheter length and providing relatively large and stable distal end fluid outlets for the lumens 14. Again, because of its flared configuration for each of the catheter tube lumens 14, the distal portion 30 of the catheter tube 12 exhibits relatively larger values for both the areal size A of the lumens and the area moment of inertia I. The number, size, and placement of the flared segments can vary according to application or catheter configuration.

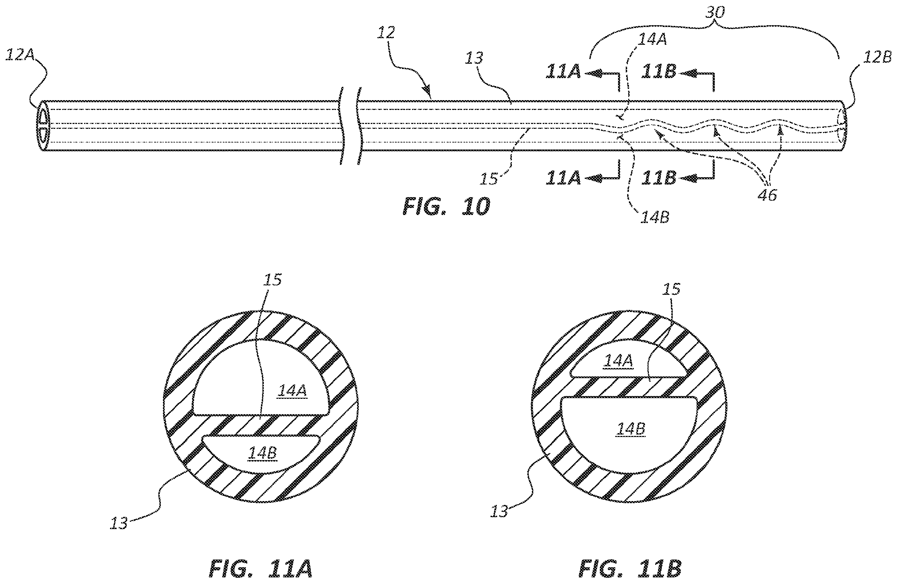

FIG. 10 shows details of the catheter tube 12 according to another non-claimed example, wherein the tube defines first and second lumens 14A and 14B separated by the septum 15. As shown, the outer wall 13 of the catheter tube 12 remains cylindrical while the septum 15 in the distal portion 30 includes a plurality of wave formations 46 that together define an undulating pattern. The distal portion 30 of the catheter tube 12 here is trimmable such that the lumen size configuration at the trimmed distal tip can be specifically selected. This shown in FIGS. 11A and 11B, wherein if the catheter tube 12 is trimmed at the location indicated at 11A - 11A in FIG. 10, the lumen 14A will be larger in area relative to the lumen 14B at the distal tip of the tube (FIG. 11A).

In contrast, trimming the catheter tube 12 at the location indicated at 11B - 11B in FIG. 10 will result in the distal tip lumen configuration shown in FIG. 11B, wherein the lumen 14B is larger in area relative to the lumen 14A. In this way, a particular lumen 14 of the catheter tube 12 can be selected to have a relatively larger distal tip opening in order to stabilize the distal portion 30 within the vessel when fluid is infused into the vessel from that lumen. Thus, it is seen that the undulating septum causes the cross sectional sizes of the lumens 14A and 14B to vary inversely with respect to one another as a function of length along the catheter tube 12. Of course, the catheter tube here can also be employed without first being trimmed.

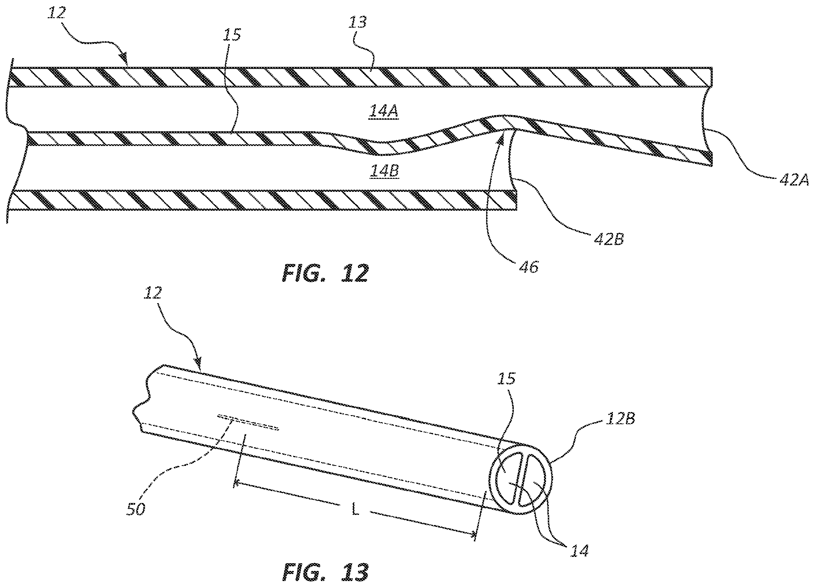

FIG. 12 shows another non-claimed distal tip trimming configuration for the catheter tube of FIG. 10, wherein the tube is trimmed to define a staggered distal tip. Specifically, the distal tip is trimmed with respect to the septum wave formations 46 such that the areal size of the tip opening 42A and 42B for each lumen 14A and 14B, respectively, is maximized. This results in enhanced stability of the distal portion of the catheter tube 12 within the patient's vessel during fluid infusion due to the increase in lumen area A at the catheter tube distal tip. Of course, other staggered tip configurations can be employed.

FIG. 13 shows details of a dual lumen catheter tube 12 according to another non-claimed example, wherein the dual lumens 14 of the tube are separated by the septum 15. At a predetermined distance L from the distal end 12B of the catheter tube 12, a slit 50 is defined in the septum 15 such that fluid communication is established between the lumens 14. So configured, the slit 50 enables the passage of fluid from one lumen 14 to the other lumen during fluid infusion into the vessel of the patient in which the catheter tube 12 is disposed. This in turn lowers the fluid pressure in the lumen 14 from which fluid is passing through the slit 50 and increases usable lumen area through which the fluid may pass from the catheter tube 12 into the vessel. These effects contribute to increase tip stability and to prevent distal tip whipping within the vessel. Note that the size, shape, number, positioning, and other variations of the slit can change according to other embodiments.

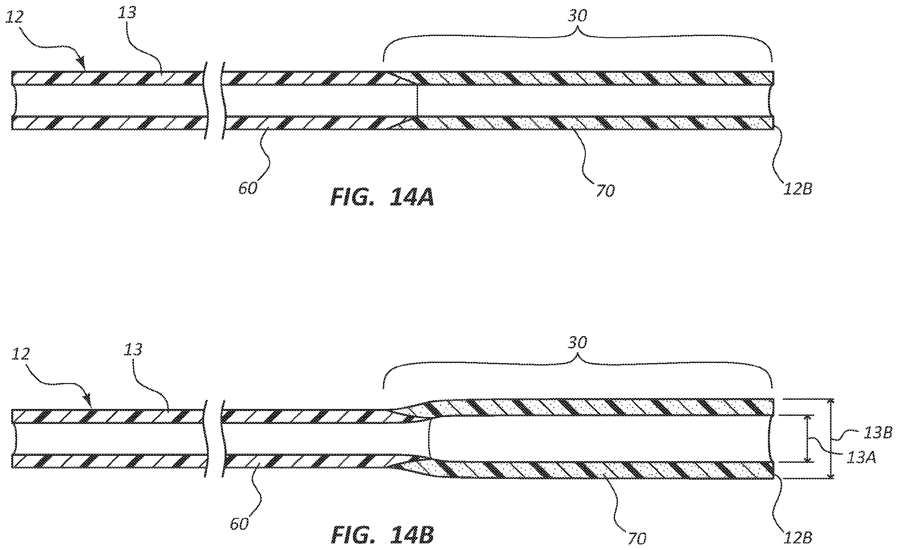

FIGS. 14A and 14B show details of the catheter tube 12 according to another embodiment, wherein a proximal portion of the catheter tube outer wall 13 includes a non-swellable first material 60. The distal portion 30 of the outer wall 13 includes a second material 70 that is configured to define a similar form factor to the proximal portions of the catheter tube including the first material 60 before insertion into the patient's body. In contrast to the first material 60, however, the second material 70 is configured to swell to a larger size when subjected to body heat or moisture after insertion into the vasculature of the patient.

So configured, the distal portion 30 of the catheter tube 12 initially defines a similar outer diameter as that of the more proximal tube portion (FIG. 14A), thus facilitating relative ease in inserting the catheter tube into the patient vasculature. After placement is complete, the second material 70 of the distal portion 30 swells (FIG. 14B) to a larger inner diameter 13A and outer diameter 13B relative to the more proximal catheter tube portion so as to provide a larger lumen area and outer wall thickness, which cooperate to increase distal tip stability. One example of a swellable material for use as the second material 70 is a biomaterial including polyurethane and polyethylene oxide sold under the trademark AQUAVENE of Pierre Fabre Dermo-Cosmetique. Other swelling materials, such as suitable hydratable and hydrophilic materials can also be employed. Use of a swelling material therefore serves as another example, in addition to the other embodiments described herein, for providing a catheter tube with a stable distal portion.

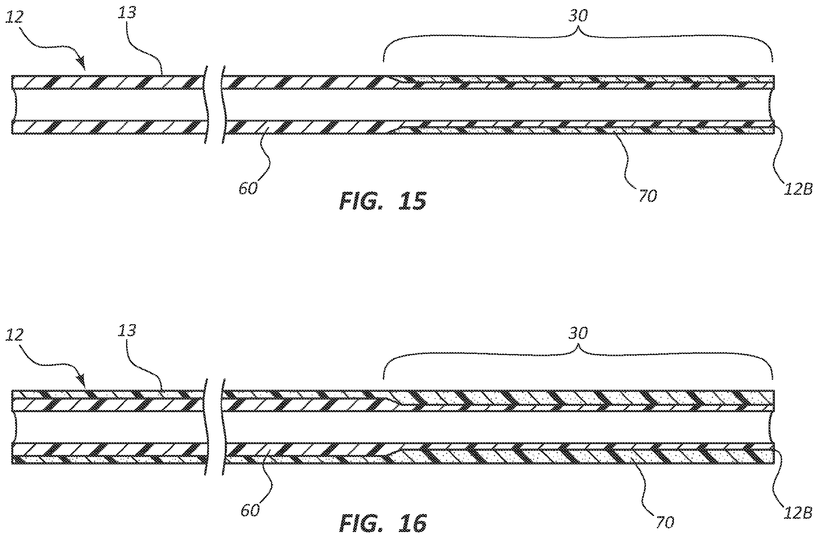

FIGS. 15 and 16 depict other configurations for use of the swellable material 70 described above in connection with FIGS. 14A and 14B. In particular, FIG. 15 shows inclusion of the swellable second material 70 in the catheter tube 12 to define only an external tubular portion of the distal portion 30 of the catheter tube 12. The internal tubular portion of the distal portion 30, as well as the more proximal portions of the catheter tube 12, is defined by the non-swelling first material 60.

In FIG. 16, the swellable second material 70 extends proximally beyond the distal portion 30 into more proximal portions of the catheter tube 12 to define at least a portion of the outer surface of the catheter tube. A catheter tube manufactured in this fashion can be designed so as to vary in a predetermined and controlled manner the degree of swelling of the catheter tube along the length thereof after insertion into the patient by controlling the amount of swellable material included in the outer wall along the tube length. It is noted that where a hydrophilic material is included in the swellable material and defines the catheter tube in a manner similar to that shown in FIG. 16, the hydrophilic material can improve the biocompatibility of the catheter tube in certain circumstances. Further note that the embodiments of FIGS. 15 and 16 isolate the swellable material 70 from direct contact with fluids passing through the lumen of the catheter tube for infusion into the patient, thus preventing unintended absorption of the fluids by the swellable material. It is appreciated that the above catheter tube configurations can be formed via extrusion or other suitable method. Also note that these configurations are just examples of many possible catheter tube designs including swellable and non-swellable materials.

Note that though the distal portions of the catheter tubes described above include both increased outer wall thickness and increased lumen area relative to the more proximal portions of the catheter tubes, in other embodiments the outer wall thickness can vary independently of lumen area in the distal portion, and vice versa. Further, the length and relative size of the distal, proximal, and portions of the catheter tube can also vary from what is shown and described herein. Note also that various single and dual lumen catheters are described herein, but other multi-lumen catheters and tubular indwelling medical devices can also benefit from the teachings herein.

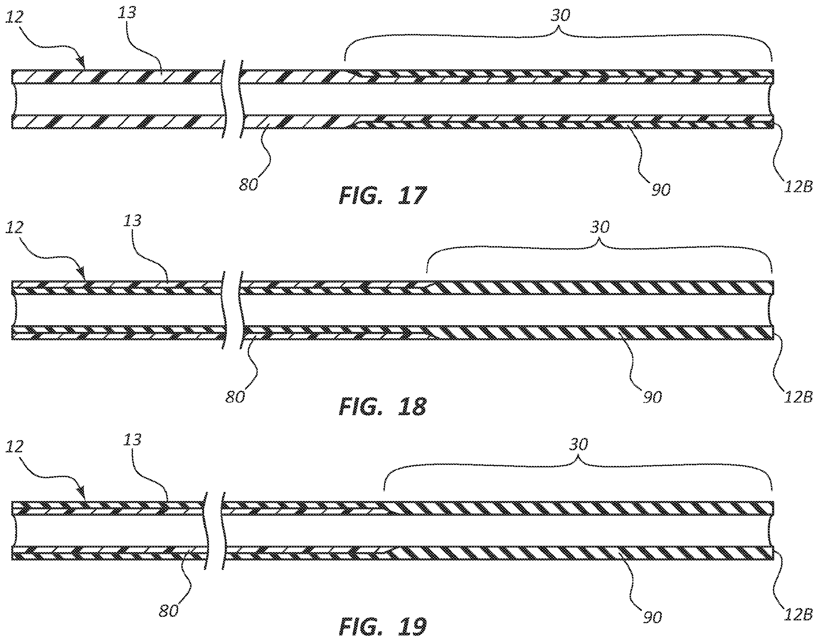

FIGS. 17-19 show details of the catheter tube 12 according to non-claimed examples, wherein the catheter tube includes a multi-durometer configuration. In particular, FIG. 17 shows the catheter tube 12 formed by the outer wall 13, a proximal portion of which includes a relatively hard (high durometer) first material 80. The first material 80 extends distally to the distal end 12B of the catheter tube 12 to define an inner diameter in the distal portion 30 of the tube. A second material 90 that is softer (low durometer) relative the first material 80 is included atop the first material in the distal portion 30 to define an outer diameter surface of the distal portion. Such a multi-durometer construction can be achieved via a selective extrusion/coextrusion process, extrusion of the first material 80 followed by coating or other application of the second material 90, etc. These and other manufacturing methods are contemplated for this and the other embodiments depicted in the succeeding figures.

The design of the catheter tube 12 as shown in FIG. 17 enables the distal portion 30, which is inserted into the patient's vasculature, to be relatively soft so as to enable the catheter tube to bend and be readily positioned within a vessel without causing trauma to the vessel. In contrast, the more proximal portion of the catheter tube 12 formed from the first material 80 remains external to the patient in the present embodiment and is relatively harder with respect the second material 90 so as to enable it to withstand the relatively higher fluid pressures present in the proximal portion of the catheter tube when fluid is infused therethrough.

FIG. 18 shows another non-claimed catheter tube example, wherein the distal portion of the catheter tube 12 is formed exclusively from the relatively soft second material 90. The second material 90 also extends proximally from the distal portion 30 of the catheter tube 12 to define an inner diameter of the more proximal portions of the tube, while the relatively harder first material 80 extends proximally from the distal portion 30 to define an outer surface of the catheter tube, as shown. Such a configuration increases the softness and flexibility of the catheter tube 12 along its entire length, with substantial softness along its distal portion 30.

In FIG. 19, another non-claimed catheter tube example is shown, wherein the distal portion of the catheter tube 12 is again formed exclusively from the relatively soft second material 90. The second material 90 also extends proximally from the distal portion 30 of the catheter tube 12 to define an outer surface of the more proximal portion of the tube, while the relatively harder first material 80 extends proximally from the distal portion 30 to define an inner diameter of the catheter tube, as shown. As with the previous two embodiments, such a configuration increases the softness and flexibility of the catheter tube 12 along its entire length, with substantial softness along its distal portion 30.

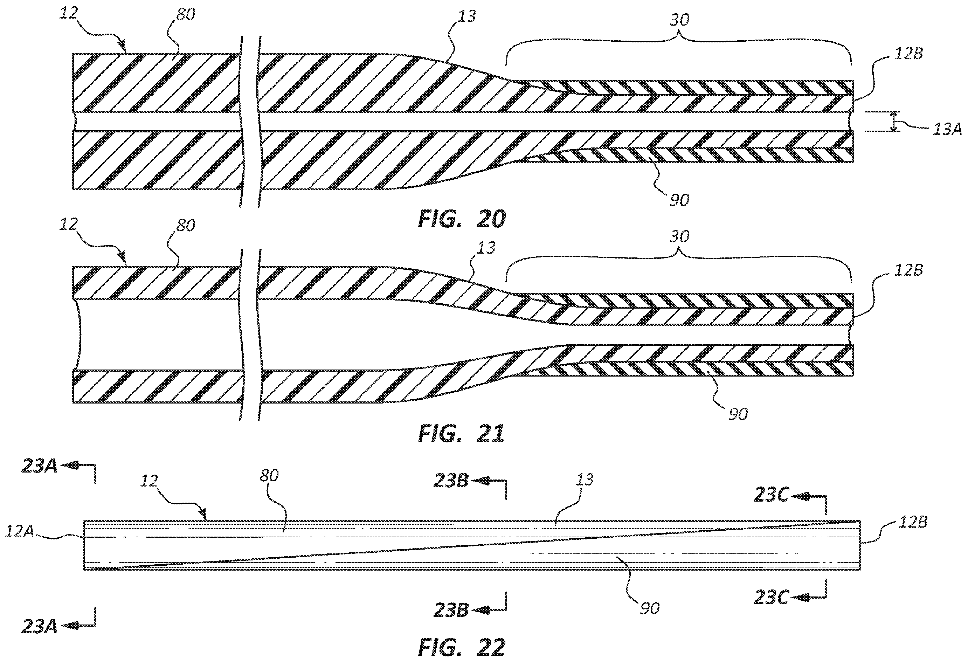

FIGS. 20 and 21 show that, in other non-claimed examples, the multi-durometer catheter tube can also vary in diameter. For instance, FIG. 20 shows the distal portion 30 of the catheter tube 12 as including an outer portion of the outer wall 13 formed by the second material 90 and the inner diameter 13A defined by the first material 80. The first material 80 extends proximally from the distal portion 30 to define the more proximal portion of the tube outer wall 13, thus defining the uniformly sized inner diameter 13A along the entire length of the catheter tube 12. The more proximal portion of the catheter tube 12 defined by the first material 80 is also sized to a larger diameter than the distal portion 30. FIG. 21 shows a similar configuration as that of FIG. 20, with the first material 80 defining a varying inner diameter for the catheter tube 12. Thus, these embodiments illustrate the many variations possible with multi-durometer catheter tube combinations. It should be appreciated that various other configurations are possible, including reverse configurations of those illustrated and described herein. Non-limiting examples of materials that may be employed include the thermoplastics mentioned further above including an 80-95A durometer range for the second material 90, and an 95A-60D durometer range for the first material 80.

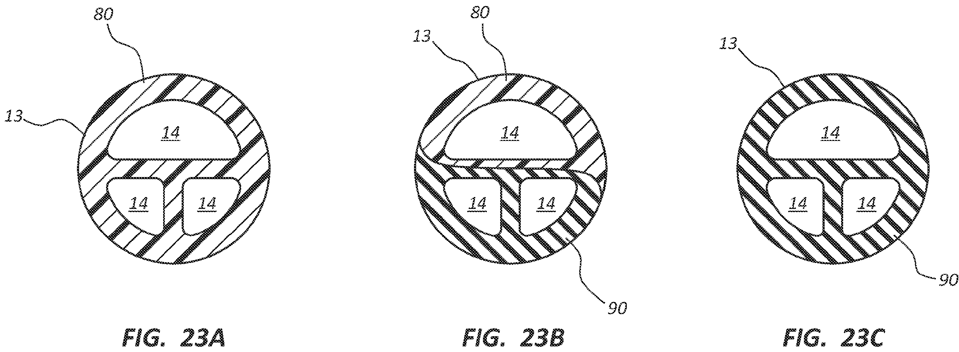

FIG. 22 shows another multi-durometer catheter tube 12 according to one non-claimed example, including the outer wall 13 defined by both the relatively hard first material 80 and the relatively soft second material 90. As shown, the proximal end 12A of the catheter tube 12 is defined completely by the first material 80, which tapers down gradually in the distal direction so as to define an increasingly smaller portion of the outer wall 13 (and septum, if present). Correspondingly, the second material 90 defines a small portion of the catheter tube 12 proximate the proximal end 12A and tapers up gradually in the distal direction so as to define an increasingly greater portion of the outer wall 13 (and septum, if present) until at the distal end 12B the entirety of the catheter tube is defined entirely by the second material 90. This varying definition of the catheter tube outer wall can be seen in the cross sectional views of FIGS. 23A-23C, which are views at the corresponding indicated locations along the catheter tube shown in FIG. 22. Thus, each point along the length of the catheter tube 12 include a unique proportion of contribution to the outer wall composition by the first material 80 and the second material 90. Such a catheter tube as shown at 12 here can be manufactured using a co-extrusion or other suitable process.

Definition of the catheter tube 12 in the manner shown in FIG. 22 enables the distal portion of the tube to be substantially softer relative more proximal portions of the tube, which is useful for providing less traumatic insertion of the tube into the vessel of the patient while still providing a relatively hard proximal portion for withstanding the relatively greater pressures present in the proximal tube portion during power injection or other fluid infusion procedures. It is appreciated that variations to this design can be employed, including the contribution to the outer wall composition being varied in a step-wise fashion as opposed to the continuous fashion shown in FIG. 22, reversal of the first and second materials, etc.

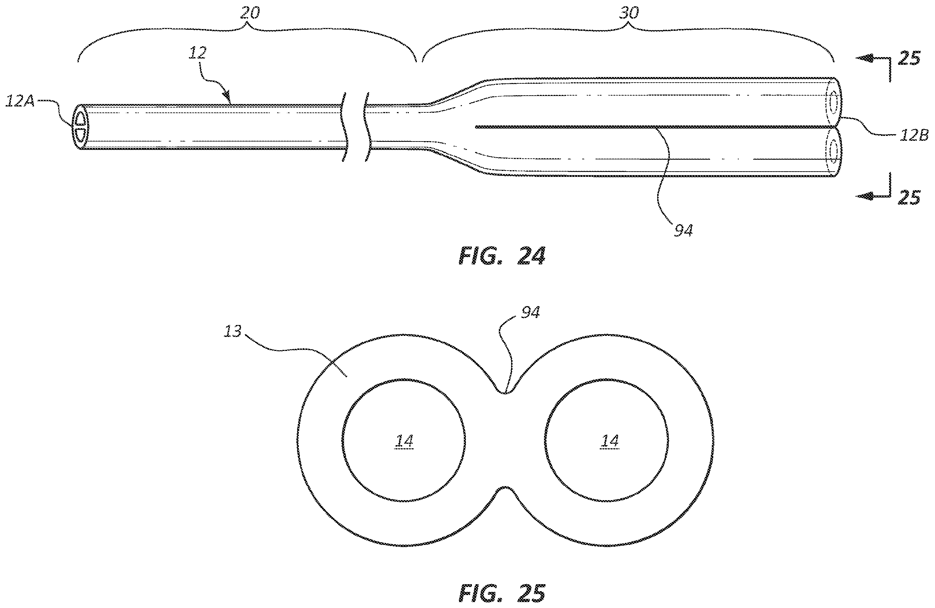

FIG. 24 gives various details of another configuration of the catheter tube 12 according to one claimed embodiment, wherein the tube is dual lumen and the distal portion 30 is flared to define a diameter greater relative to that of the proximal portion 20. The tube 12 in the distal portion 30 includes two single lumen tube structures, each defining one of the lumens 14, which are joined via a crease 94 therebetween, best seen in FIG. 25. This configuration offers the same benefits of a flared distal portion as in previous embodiments, e.g., reduction in distal tip whipping within the vessel.

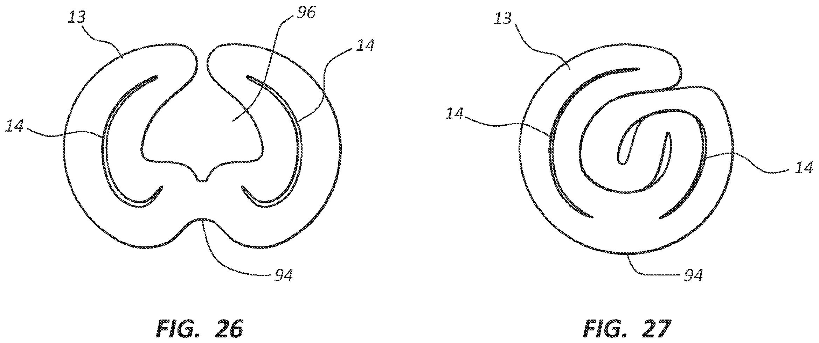

FIGS. 26 and 27 show that the outer wall 13 that defines the distal portion 30 of the catheter tube 12 can be folded or compressed in order to substantially match in diameter the diameter of the proximal portion 20. This enables the catheter tube 12 to be fed through an introducer and into the patient vessel. After placement of the catheter tube 12 within the vessel is complete, the distal portion can unfold to its full size shown in FIG. 25.

For instance, FIG. 26 shows that the outer wall 13 defining the distal portion 30 of the catheter tube 12 can be compressed so as to close the lumens 14 and define a substantially closed, longitudinally extending cavity 96. The cavity 96 is suitable in one embodiment for receiving therethrough a guidewire for guiding the catheter tube into the vessel. A dissolvable adhesive or other suitable substance can be applied to the catheter tube outer wall 13 so as to maintain the tube in the compressed state until placement within the patient's vasculature is complete. The adhesive can then dissolve, enabling the distal portion 30 of the catheter tube 12 to expand to its fully open state.

FIG. 27 shows the outer wall 13 in a rolled configuration that enables the distal portion 30 of the catheter tube 12 to assume a diameter substantially similar to that of the proximal portion 20 so as to ease catheter tube insertion into the patient's vasculature. Again, a dissolvable adhesive or other suitable substance can be employed to maintain the distal portion outer wall 13 in the rolled configuration during insertion. In addition to the configurations shown in FIGS. 26 and 27, other compression configurations are also possible. Also, catheter tubes defining more or fewer than two lumens can also benefit from the principles described herein. In the illustrated and other embodiments described herein, it is further appreciated that the cross sectional geometry can also vary from proximal to distal ends, such as from round to oval, in one non-limiting example.

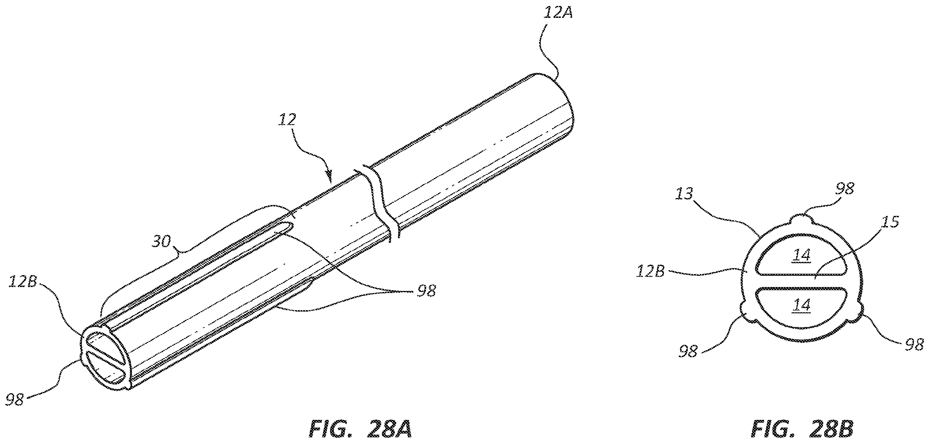

FIGS. 28A and 28B depict yet another stable configuration for the distal portion 30 of the catheter tube 12, wherein a plurality of longitudinally extending stiffening ribs 98 are disposed on the outer wall 13. The ribs 98 serve to increase the area moment of inertia I for the catheter tube distal portion 30, thus increasing tube stability during fluid infusion therethrough and reducing or preventing distal tip whipping. Though three ribs are shown disposed about the circumference of the catheter tube 12, more or fewer than this can be employed. Also, the size, shape, length, and placement of the ribs can vary from what is shown in FIGS. 28A and 28B. In one claimed embodiment, the ribs can be disposed on an inner lumen surface of the distal portion.

In addition to the catheter tubes described herein as part of catheter assemblies, the principles disclosed can be employed with other tubular medical devices as well.

The described embodiments are to be considered in all respects only as illustrative, not restrictive. The scope of the embodiments is, therefore, indicated by the appended claims rather than by the foregoing description.

## Claims

1. A catheter assembly (10), comprising:
a catheter tube (12) for insertion into a vessel of a patient, the catheter tube defining at least one lumen (14) and configured to withstand power injection, the catheter tube including:
a proximal portion (20) at least a portion of which defines a first cross sectional size that is sized to reside in a relatively distal portion of the vessel; and a distal portion (30) extending distally from the distal end of the proximal portion to the distal end of the tube, the distal portion flared in size from that of the proximal portion and defining a second cross sectional size that is sized larger relative the first cross sectional size so as to prevent whipping of a distal tip of the catheter tube positioned at a target position within the vessel during infusion of a fluid through the catheter tube into the vessel, **characterised in that**
the thickness of the outer wall (13) of the distal portion of the catheter tube is greater than the thickness of the outer wall (13) of the proximal portion of the catheter tube.

2. The catheter assembly as defined in claim 1, wherein the cross sectional area of the at least one lumen (14) of the catheter tube is larger in the distal portion of the catheter tube than in the proximal portion of the catheter tube.

3. The catheter assembly as defined in claim 1, wherein the cross sectional area of the at least one lumen of the catheter tube is the same in the distal portion of the catheter tube as in the proximal portion of the catheter tube.

4. The catheter assembly as defined in claim 1, wherein the catheter tube defines at least two lumens (14A, 14B) and is distally trimmable, the at least two lumens defining a cross sectional area that is larger in the distal portion than the cross sectional area of the lumens in the portion of the proximal portion defining the first cross sectional size.

5. The catheter assembly as defined in claim 1, wherein the entirety of the distal portion defines the second cross sectional size.

6. The catheter assembly as defined in claim 1, wherein the distal portion includes a distal taper region (34, 35) increasing the size of the catheter tube from the first cross sectional size to the second cross sectional size.

7. The catheter assembly as defined in claim 6, wherein the proximal portion includes a proximal taper region (24) proximate a proximal end (12A) of the catheter tube, the proximal taper region decreasing the size of the catheter tube from a third cross sectional size to the first cross sectional size.

8. The catheter assembly as defined in any of claims 1 to 7, wherein the proximal portion defining the first cross sectional size includes an elastic modulus that is lower relative to an elastic modulus of the distal portion defining the second cross sectional size.

## Patentansprüche

1. Katheterbausatz (10), umfassend:
ein Katheterrohr (12) zum Einsatz in ein Gefäßsystem eines Patienten, wobei das Katheterrohr zumindest ein Lumen (14) definiert und dazu ausgebildet ist, einer Kraftinjektion zu widerstehen, wobei das Katheterrohr einschließt:
einen proximalen Abschnitt (20), bei dem zumindest ein Abschnitt davon eine erste Querschnittsgröße definiert, die dafür bemessen ist, in einem relativ distalen Abschnitt des Gefäßes zu liegen; und einen distalen Abschnitt (30), der sich distal von dem distalen Ende des proximalen Abschnitts zu dem distalen Ende des Rohrs erstreckt, wobei die Größe des distalen Abschnitts von jener des proximalen Abschnitts ausgehend aufgeweitet ist und eine zweite Querschnittsgröße definiert, die relativ zu der ersten Querschnittsgröße größer bemessen ist, um ein Ausschlagen einer distalen Spitze des Katheterrohrs, die an einer Zielposition innerhalb des Gefäßes positioniert ist, während einer Infusion eines Fluids durch das Katheterrohr in das Gefäß zu verhindern, **dadurch gekennzeichnet, dass**
die Dicke der Außenwand (13) des distalen Abschnitts des Katheterrohrs größer ist als die Dicke der Außenwand (13) des proximalen Abschnitts des Katheterrohrs.

2. Katheterbausatz nach Anspruch 1, wobei die Querschnittsfläche des zumindest einen Lumens (14) des Katheterrohrs in dem distalen Abschnitt des Katheterrohrs größer ist als in dem proximalen Abschnitt des Katheterrohrs.

3. Katheterbausatz nach Anspruch 1, wobei die Querschnittsfläche des zumindest einen Lumens des Katheterrohrs in dem distalen Abschnitt des Katheterrohrs und in dem proximalen Abschnitt des Katheterrohrs gleich ist.

4. Katheterbausatz nach Anspruch 1, wobei das Katheterrohr zumindest zwei Lumina (14A, 14B) definiert und distal kürzbar ist, wobei die zumindest zwei Lumina eine Querschnittsfläche definieren, die in dem distalen Abschnitt größer ist als die Querschnittsfläche der Lumina in dem Abschnitt des proximalen Abschnitts, der die erste Querschnittsgröße definiert.

5. Katheterbausatz nach Anspruch 1, wobei die Gesamtheit des distalen Abschnitts die zweite Querschnittsgröße definiert.

6. Katheterbausatz nach Anspruch 1, wobei der distale Abschnitt einen distalen Verjüngungsbereich (34, 35) einschließt, in dem die Größe des Katheterrohrs von der ersten Querschnittsgröße auf die zweite Querschnittsgröße zunimmt.

7. Katheterbausatz nach Anspruch 6, wobei der proximale Abschnitt einen proximalen Verjüngungsbereich (24) in der Nähe eines proximalen Endes (12A) des Katheterrohrs einschließt, wobei in dem proximalen Verjüngungsbereich die Größe des Katheterrohrs von einer dritten Querschnittsgröße auf die erste Querschnittsgröße abnimmt.

8. Katheterbausatz nach einem der Ansprüche 1 bis 7, wobei der proximale Abschnitt, der die erste Querschnittsgröße definiert, einen Elastizitätsmodul einschließt, der relativ kleiner ist als ein Elastizitätsmodul des distalen Abschnitts, der die zweite Querschnittsgröße definiert.

## Revendications

1. Ensemble cathéter (10) comprenant :
un tube de cathéter (12) destiné à être inséré dans un vaisseau d'un patient, le tube de cathéter définissant au moins une lumière (14) et étant configuré pour résister à une injection sous pression, le tube de cathéter incluant :
une portion proximale (20) dont au moins une portion définit une première dimension de section transversale qui est dimensionnée pour résider dans une portion relativement distale du vaisseau ; et une portion distale (30) s'étendant distalement depuis l'extrémité distale de la portion proximale jusqu'à l'extrémité distale du tube, la portion distale étant évasée en dimension par rapport à celle de la portion proximale et définissant une deuxième dimension de section transversale qui est dimensionnée comme étant plus grande par rapport à la première dimension de section transversale de manière à empêcher le fouettement d'une pointe distale du tube de cathéter positionnée à une position cible à l'intérieur du vaisseau pendant l'infusion d'un fluide à travers le tube de cathéter dans le vaisseau, **caractérisé en ce que**
l'épaisseur de la paroi externe (13) de la portion distale du tube de cathéter est supérieure à l'épaisseur de la paroi externe (13) de la portion proximale du tube de cathéter.

2. Ensemble cathéter selon la revendication 1, dans lequel l'aire de section transversale de l'au moins une lumière (14) du tube de cathéter est plus grande dans la portion distale du tube de cathéter que dans la portion proximale du tube de cathéter.

3. Ensemble cathéter selon la revendication 1, dans lequel l'aire de section transversale de l'au moins une lumière du tube de cathéter est la même dans la portion distale du tube de cathéter que dans la portion proximale du tube de cathéter.

4. Ensemble cathéter selon la revendication 1, dans lequel le tube de cathéter définit au moins deux lumières (14A, 14B) et peut être ajusté de manière distale, les au moins deux lumières définissant une aire de section transversale qui est plus grande dans la portion distale que l'aire de section transversale des lumières dans la portion de la portion proximale définissant la première dimension de section transversale.

5. Ensemble cathéter selon la revendication 1, dans lequel la totalité de la portion distale définit la deuxième dimension de section transversale.

6. Ensemble cathéter selon la revendication 1, dans lequel la portion distale inclut une région conique distale (34, 35) qui augmente la dimension du tube de cathéter depuis la première dimension de section transversale jusqu'à la deuxième dimension de section transversale.

7. Ensemble cathéter selon la revendication 6, dans lequel la portion proximale inclut une région conique proximale (24) à proximité d'une extrémité proximale (12A) du tube de cathéter, la région conique proximale diminuant la dimension du tube de cathéter depuis une troisième dimension de section transversale jusqu'à la première dimension de section transversale.

8. Ensemble cathéter selon l'une quelconque des revendications 1 à 7, dans lequel la portion proximale définissant la première dimension de section transversale inclut un module d'élasticité qui est inférieur par rapport à un module d'élasticité de la portion distale définissant la deuxième dimension de section transversale.
